# EUROPÄISCHE PATENTSCHRIFT

(11) **EP 1 132 078 B1**
(45) Veröffentlichungstag und Bekanntmachung des Hinweises auf die Patenterteilung: **17.12.2008**
(21) Anmeldenummer: 01101424.8
(22) Anmeldetag: 23.01.2001
(51) Int. Cl.: A61K 8/81, A61Q 17/04

(54) **Kosmetische oder dermatologische Lichtschutzmittelzubereitungen**
Cosmetic or dermatological sunscreens
Filtres solaires cosmétiques ou dermatologiques

(30) Priorität: 18.02.2000 DE 10007486
(43) Veröffentlichungstag der Anmeldung: 12.09.2001
(73) Patentinhaber: BASF SE, 67056 Ludwigshafen (DE)
(72) Erfinder: Hössel, Peter, Dr., 67105 Schifferstadt (DE); Wünsch, Thomas, Dr., 67346 Speyer (DE); Dieing, Reinhold, Dr., 67105 Schifferstadt (DE)

(56) Entgegenhaltungen:
- DE-A- 4 213 971
- DE-A- 19 731 764
- DE-A- 19 812 777
- US-A- 3 454 482
- US-A- 5 553 630

## Beschreibung

Die vorliegende Erfindung betrifft eine Mischung aus mindestens einem mittels Lösungspolymerisation erhältlichen Copolymeren und mindestens einem anorganischen UV-Filter und deren Verwendung zur Herstellung kosmetischer und dermatologischer Lichtschutzmittelzubereitungen.

Die in kosmetischen und dermatologischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche sowohl die UV-A-Strahlung als auch die UV-B-Strahlung absorbieren.

Dabei haben in den letzten Jahren neben organischen UV-Absorbern auch anorganische Lichtschutzmittel in der Kosmetik und Dermatologie immer mehr an Bedeutung gewonnen.

Als geeignete anorganische Lichtschutzfilter seien hier Titandioxid, Zinkoxid, Eisenoxide oder auch Ceroxid genannt.

Mikropigmente, vor allem mikronisiertes Titandioxid oder Zinkoxid, zeichnen sich durch ihre hohe Verträglichkeit und ihre besondere Stabilität aus. Über einen breiten UV-Bereich von 250 bis 380 nm kann mit Titandioxiden und/oder Zinkoxiden ein außerordentlich wirksamer Schutz erzielt werden.

Die o.g. anorganischen Filter haben jedoch den Nachteil, daß bei ihrer Verwendung zur Herstellung kosmetischer oder dermatologischer Zubereitungen häufig Dispersionsprobleme auftreten, da sich die Partikel in den kosmetischen Formulierungen oftmals absetzen und daher eine optimale Anwendung auf der Haut nicht gewährleistet ist.

Es war daher Aufgabe der vorliegenden Erfindung, neuartige kosmetische Mittel zum Schutz der Haut bereitzustellen, welche eine verbesserte Stabilität sowie gute Formulierungseigenschaften aufweisen, darüberhinaus verbesserte sensorische Eigenschaften und einen hohen Lichtschutzfaktor besitzen.

Diese Aufgabe wurde gelöst durch Mischungen, enthaltend
A) mindestens ein Copolymer, das erhältlich ist durch
   (i) radikalisch initiierte Lösungspolymerisation eines Monomerengemisches aus
      (a) 0,01 bis 99,99 Gew.-%, bevorzugt 2 bis 94,98 Gew.-%, besonders bevorzugt 10 bis 70 Gew.-% mindestens eines Monomers, ausgewählt aus der Gruppe, bestehend aus N-Vinylimidazolen und Diallylaminen, gegebenenfalls in partiell oder vollständig quaternisierter Form;
      (b) 0,01 bis 99,99 Gew.-%, bevorzugt 5 bis 97,98 Gew.-%, besonders bevorzugt 20 bis 89,95 Gew.-% mindestens eines von (a) verschiedenen neutralen oder basischen wasserlöslichen Monomers;
      (c) 0 bis 50 Gew.-%, bevorzugt 0 bis 40 Gew.-%, besonders bevorzugt 0 bis 30 Gew.-% mindestens einer ungesättigten Säure oder eines ungesättigten Anhydrids;
      (d) 0 bis 50 Gew.-%, bevorzugt 0 bis 40 Gew.-%, besonders bevorzugt 0 bis 30 Gew.-% mindestens eines weiteren von (a), (b) oder (c) verschiedenen radikalisch copolymerisierbaren Monomers; und
      (e) 0 bis 10 Gew.-%, bevorzugt 0,01 bis 10 Gew.-%, besonders bevorzugt 0,02 bis 8 Gew.-%, ganz besonders bevorzugt 0,05 bis 5 Gew.-% mindestens eines als Vernetzer wirkenden Monomers mit mindestens zwei ethylenisch ungesättigten, nichtkonjugierten Doppelbindungen und
   (ii) anschließende teilweise oder vollständige Quaternisierung oder Protonierung des Polymeren für den Fall, daß das Monomer (a) nicht oder nur partiell quaternisiert ist und
B) mindestens einen anorganischen UV-Filter.

Kationische Polymere werden bereits häufig als Konditioniermittel in haarkosmetischen Formulierungen eingesetzt. Sie bewirken in erster Linie eine Verbesserung der Naßkämmbarkeit des Haares. Außerdem verhindern kationische Polymere die elektrostatische Aufladung des Haares.

So wird z.B. in der EP-A-0 246 580 die Verwendung von unvernetzten Homo- und Copolymeren von 3-Methyl-1-vinylimidazoliumchloriden in kosmetischen Mitteln beschrieben. Die EP-A-0 544 158 und US-A-4,859,756 beanspruchen die Verwendung von unvernetzten Homo- und Copolymeren von chloridfreien, quaternisierten N-Vinylimidazolen in kosmetischen Zubereitungen. Aus der EP-A-0 715 843 ist die Verwendung von unvernetzten Copolymeren aus einem quaternisierten N-Vinylimidazol, N-Vinylcaprolactam und N-Vinylpyrrolidon sowie optional einem weiteren Comonomer in kosmetischen Zubereitungen bekannt.

EP-A-0 893 117 beschreibt die Verwendung von vernetzten kationischen Copolymeren als Konditionierungsmittel in Zubereitungen für die Haarkosmetik.

US-A-5553630 offenbart eine Mischung, die einen anorganischen UV-Filter und ein Copolymer aus Hydroxyethylcellulose mit Diallylamin-dimethyl-ammoniumchlorid enthält.

Bei der in den erfindungsgemäßen Mischungen enthaltenen Komponente A) kann es sich sowohl um unvernetzte als auch um vernetzte kationische Polymerisate handeln. Bevorzugt sind im Rahmen der vorliegenden Erfindung vernetzte kationische Copolymerisate.

Geeignete Monomere (a) sind die N-Vinylimidazol-Derivate der allgemeinen Formel (I), 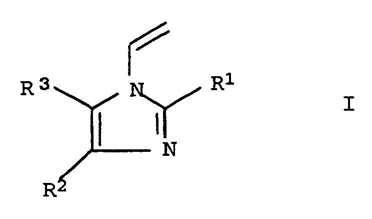 worin die Reste R¹ bis R³ unabhängig voneinander für Wasserstoff, C₁-C₄-Alkyl oder Phenyl stehen.

Weiterhin eignen sich Diallyamine der allgemeinen Formel (II), 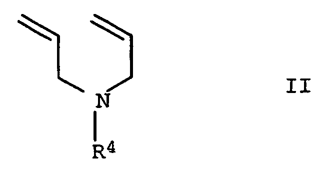 worin R⁴ für C₁-C₂₄-Alkyl steht.

Beispiele für Verbindungen der allgemeinen Formel (I) sind folgender Tabelle 1 zu entnehmen:

**Tabelle 1**

| R¹ | R² | R³ |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Weitere brauchbare Monomere der Formel (I) sind die Ethyl-, Propyl- oder Butyl-Analoga der in Tabelle 1 aufgelisteten Methylsubstituierten 1-Vinylimidazole.

Beispiele für Verbindungen der allgemeinen Formel (II) sind Diallylamine, worin R⁴ für Methyl, Ethyl, iso- oder n-Propyl, iso-, n- oder tert.-Butyl, Pentyl, Hexyl, Heptyl, Octyl, Nonyl oder Decyl steht. Beispiele für längerkettige Reste R⁴ sind Undecyl, Dodecyl, Tridecyl, Pentadecyl, Octadecyl und Icosayl.

Die Monomere (a) können entweder in quaternisierter Form als Monomere eingesetzt werden oder nicht-quaternisiert polymerisiert werden, wobei man im letzteren Fall das erhaltene Copolymer entweder quaternisiert oder protoniert.

Zur Quaternisierung der Verbindungen der allgemeinen Formeln (I) und (II) eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternisierung der basischen Monomere der allgemeinen Formel (I) und (II) kann auch mit Alkylenoxiden wie Ethylenoxid oder Propylenoxid in Gegenwart von Säuren durchgeführt werden.

Die Quaternisierung des Monomeren oder eines Polymeren mit einem der genannten Quaternisierungsmittel kann nach allgemein bekannten Methoden erfolgen.

Die Quaternisierung des Copolymeren kann vollständig oder auch nur teilweise erfolgen. Der Anteil quaternisierter Monomere (a) im Copolymeren kann über einen weiten Bereich variieren und liegt z.B. bei etwa 20 bis 100 Mol.-%.

Bevorzugte Quaternierungsmittel sind Methylchlorid, Dimethylsulfat oder Diethylsulfat.

Bevorzugte Beispiele für Monomere (a) sind 3-Methyl-1-vinylimidazoliumchlorid und -methosulfat, Dimethyldiallylammoniumchlorid.

Besonders bevorzugte Monomere (a) sind 3-Methyl-1-vinylimidazoliumchlorid und -methosulfat.

Zur Protonierung eignen sich beispielsweise Mineralsäuren wie HCl, H₂SO₄, H₃PO₄, sowie Monocarbonsäuren, wie z.B. Ameisensäure und Essigsäure, Dicarbonsäuren und mehrfunktionelle Carbonsäuren, wie z.B. Oxalsäure und Zitronensäure, sowie alle anderen protonenabgebenden Verbindungen und Substanzen, die in der Lage sind, das entsprechende Vinylimidazol oder Diallylamin zu protonieren. Insbesondere eignen sich wasserlösliche Säuren zur Protonierung.

Die Protonierung des Polymers kann entweder im Anschluß an die Polymerisation erfolgen oder bei der Zubereitung der Mischung, bei der in der Regel ein physiologisch verträglicher pH-Wert eingestellt wird.

Unter Protonierung ist zu verstehen, daß mindestens ein Teil der protonierbaren Gruppen des Polymers, bevorzugt 20 bis 100 Mol-%, protoniert wird, so daß eine kationische Gesamtladung des Polymers resultiert.

Geeignete von (a) verschiedene Monomere (b) sind N-Vinyllactame, wie z.B. N-Vinylpiperidon, N-Vinylpyrrolidon und N-Vinylcaprolactam, N-Vinylacetamid, N-Methyl-N-vinylacetamid, Acrylamid, Methacrylamid, N,N-Dimethylacrylamid, N-Methylolmethacrylamid, N-Vinyloxazolidon, N-Vinyltriazol, Hydroxyalkyl(meth)acrylate, wie z.B. Hydroxyethyl(meth)acrylat und Hydroxypropyl(meth)acrylate, oder Alkylethylenglykol(meth)acrylate mit 1 bis 50 Ethylenglykol-einheiten im Molekül. Außerdem eignen sich Dialkylaminoalkyl(meth)acrylate und Dialkylaminoalkyl(meth)acrylamide, wie z.B. N,N'-Dimethylaminoethylmethacrylat oder N-[3-(Dimethylamino)propyl]methacrylamid.

Bevorzugt werden als Monomere (b) N-Vinyllactame eingesetzt. Ganz besonders bevorzugt ist N-Vinylpyrrolidon.

Als Monomere (c) eignen sich ungesättigte Carbonsäuren und ungesättigte Anhydride, wie z.B. Acrylsäure, Methacrylsäure, Crotonsäure, Itaconsäure, Maleinsäure, Fumarsäure oder ihre entsprechenden Anhydride, ungesättigte Sulfonsäuren, beispielsweise Acrylamidomethylpropansulfonsäure, sowie die Salze der ungesättigten Säuren, wie z.B. die Alkali- oder Ammoniumsalze.

Als Monomere (d) eignen sich C₁-C₄₀-Alkylester der (Meth)acrylsäure, wobei die Ester abgeleitet werden von linearen, verzweigtkettigen oder carbocyclischen Alkoholen, z.B. Methyl(meth)acrylat, Ethyl(meth)acrylat, tert.-Butyl(meth)acrylat, Isobutyl(meth)acrylat, n-Butyl(meth)acrylat, Stearyl(meth)acrylat, oder Ester von alkoxylierten Fettalkoholen, z.B. C₁-C₄₀-Fettalkoholen, umgesetzt mit Ethylenoxid, Propylenoxid oder Butylenoxid, insbesondere C₁₀-C₁₈-Fettalkohole, umgesetzt mit 3 bis 150 Ethylenoxideinheiten. Weiterhin eignen sich N-Alkyl-substituierte Acrylamide mit linearen, verzweigtkettigen oder carbocyclischen Alkylresten wie N-tert.-Butylacrylamid, N-Butylacrylamid, N-Octylacrylamid, N-tert.-Octylacrylamid.

Ferner eignen sich Styrol, Vinyl- und Allylester von C₁-C₄₀-Carbonsäuren, die linear, verzweigtkettig oder carbocyclisch sein können, z.B. Vinylacetat, Vinylpropionat, Vinylneononanoat, Vinylneoundekansäure, t-Butyl-benzoesäurevinylester, Alkylvinyl-ether, beispielsweise Methylvinylether, Ethylvinylether, Butylvinylether, Stearylvinylether.

Acrylamide, wie N-tert.-Butylacrylamid, N-Butylacrylamid, N-Octylacrylamid, N-tert.-Octylacrylamid und N-Alkyl-substituierte Acrylamide mit linearen, verzweigtkettigen oder carbocyclischen Alkylresten, wobei der Alkylrest die oben für R⁴ angegebenen Bedeutungen besitzen kann.

Monomere (e), die eine vernetzende Funktion besitzen, sind Verbindungen mit mindestens 2 ethylenisch ungesättigten, nichtkonjugierten Doppelbindungen im Molekül.

Geeignete Vernetzer sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole, wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglycolmonoester, 2,2-Bis(4-hydroxyphenyl)propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10 000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C₃- bis C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Monomere (e) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20 000.

Als Vernetzer sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z.B. Triallylmethylammoniumchlorid oder -methylsulfat, als Vernetzer geeignet.

Geeignete Vernetzter sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff.

Weitere geeignete Vernetzer sind Divinyldioxan, Tetraallylsilan oder Tetravinylsilan.

Vorzugsweise werden solche Vernetzer eingesetzt, die in der Monomermischung löslich sind.

Besonders bevorzugt eingesetzte Vernetzer sind beispielsweise Pentaerythrittriallylether, Methylenbisacrylamid, Triallylamin und Triallylalkylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer sind Pentaerythrittriallylether, Methylenbisacrylamid, N,N'-Divinylethylenharnstoff, Triallylamin und Acrylsäureester von Glykol, Butandiol, Trimethylolpropan oder Glycerin oder Acrylsäureester von mit Ethylenoxid und/oder Epichlorhydrin umgesetzten Glykol, Butandiol, Trimethylolpropan oder Glycerin.

Die Monomere (a) bis (e) können jeweils einzeln oder im Gemisch mit anderen Monomeren der gleichen Gruppe eingesetzt werden.

Die Herstellung der Polymerisate erfolgt nach den an sich bekannten Verfahren der radikalisch initiierten Lösungspolymerisation, bevorzugt in wäßrigen Medien, besonders bevorzugt in Wasser ohne Zusatz eines weiteren Lösungsmittels

Die Polymerisation erfolgt üblicherweise bei Temperaturen von 20°C bis 150°C und bei Normaldruck oder unter Eigendruck; die Temperatur kann konstant gehalten oder kontinuierlich oder diskontinuierlich erhöht werden, z.B. um den Umsatz zu steigern.

Als Initiatoren für die radikalische Polymerisation können die hierfür üblichen wasserlöslichen und wasserunlöslichen Peroxo- und/oder Azo-Verbindungen eingesetzt werden, beispielsweise Alkali- oder Ammoniumperoxidisulfate, Dibenzoylperoxid, tert.-Butylperpivalat, tert.-Butyl-per-2-ethylhexanoat, Di-tert.-butylperoxid, tert.-Butylhydroperoxid, Azo-bis-isobutyronitril, Azo-bis-(2-amidinopropan)dihydrochlorid oder 2,2'-Azo-bis-(2-methylbutyronitril). Geeignet sind auch Initiatormischungen oder Redox-Initiator Systeme, wie z.B. Ascorbinsäure/Eisen(II)sulfat/Natriumperoxodisulfat, tert.-Butylhydroperoxid /Natriumdisulfit, tert.-Butylhydroperoxid/ Natriumhydroxymethansulfinat. Die Initiatoren können in den üblichen Mengen eingesetzt werden, beispielsweise 0,05 bis 5 Gew.-%, oder 0,05 bis 0,3 Mol-%, bezogen auf die Menge der zu polymerisierenden Monomeren.

Man erhält nach der Polymerisation eine Lösung mit einem Feststoffgehalt von 5 bis 40 Gew.-%, bevorzugt von 5 bis 35 Gew.-%, besonders bevorzugt von 7 bis 20 Gew.%. Zur Erhöhung des Feststoffgehaltes können die Lösung durch Destillation teilweise oder vollständig entwässert werden.

Die hergestellten vernetzten oder unvernetzten Polymerisate können direkt in hautkosmetischen oder dermatologischen Anwendungen eingesetzt werden. Die Polymerisate werden nicht isoliert, sondern werden bevorzugt in Form ihrer Lösung direkt eingesetzt.

Das Molekulargewicht und der K-Wert der erfindungsgemäß verwendeten Copolymerisate läßt sich in an sich bekannter Weise durch die Wahl der Polymerisationsbedingungen, beispielsweise Polymerisationsdauer, Polymerisationstemperatur oder Initiatorkonzentration, und durch den Gehalt an Vernetzer in einem breiten Bereich variieren. Die K-Werte bevorzugter Copolymerisate liegen in einem Bereich zwischen 30 bis 350, vorzugsweise 50 bis 350.

Die K-Werte werden nach Fikentscher, Cellulosechemie, Bd. 13, S. 58-64 (1932) bei 25°C 0,1%ig in 0,5 molarer Kochsalzlösung gemessen.

Bei hohen Vernetzungsgraden sind die K-Werte der Polymere nicht bestimmbar.

Die in den erfindungsgemäßen Mischungen verwendeten anorganischen UV-Filter sind z.B. anorganische Pigmente auf Basis von Metalloxiden und/oder anderen in Wasser schwerlöslichen oder unlöslichen Metallverbindungen, bevorzugt der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Besonders bevorzugt handelt es sich hierbei um Pigmente auf der Basis von TiO₂ oder ZnO, ganz besonders bevorzugt auf Basis von ZnO, insbesondere ZnO-Partikel gemäß EP-A-585 239 mit einem mittleren Teilchendurchmesser von weniger als 0,2 µm, die weniger als 20 ppm Blei, weniger als 3 ppm Arsen, weniger als 15 ppm Cadmium und weniger als 1 ppm Quecksilber enthalten.

Die anorganischen Pigmente liegen vorteilhafterweise in hydrophober Form vor, d.h. daß sie oberfächlich wasserabweisend behandelt sind. Diese Oberflächenbehandlung kann darin bestehen, daß die Pigmente nach an sich bekannten Verfahren mit einer dünnen hydrophoben Schicht, insbesondere einer Silikonschicht versehen werden.

Eines solcher Verfahren besteht beispielsweise darin, daß die hydrophobe Oberflächenschicht nach der Reaktion gemäß n TiO₂ + m(RO)₃Si-R' → nTiO₂ (oberfl.)

erzeugt wird. n und m sind dabei nach Belieben einzusetzende stöchiometrische Parameter, R und R' die gewünschten organischeen Reste. Beispielsweise zu nennen sind die in Analogie zu DE-A-33 14 742 dargestellten hydrophobisierten Pigmente.

In einer bevorzugten Ausführungsform wird das Metalloxid mit einem Silikon der allgemeinen Formel III, 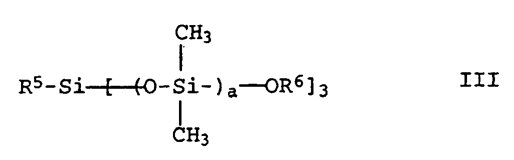 in der unabhängig voneinander R⁵ C₁-C₁₂-Alkyl, bevorzugt Octyl, Butyl oder Ethyl und R⁶ Methyl oder Ethyl bedeuten und a für Werte von 4 bis 12, bevorzugt 4 bis 8 steht, beschichtet. Hinsichtlich der Herstellung und Eigenschaften dieser Silikon-beschichteten Metalloxide sei auf US 5,756,788 verwiesen.

Der Anteil anorganischer UV-Filter in der erfindungsgemäßen Mischung beträgt 0,1 bis 99,9 Gew.-%, bevorzugt 10 bis 95 Gew.-%, besonders bevorzugt 30 bis 90 Gew.-%, bezogen auf den Feststoffgehalt der Mischung.

Es ist weiterhin vorteilhaft, der erfindungsgemäßen Mischung weitere öllösliche und/oder wasserlösliche organische UV-A- und/oder UV-B-Filter zuzusetzen, wobei die Gesamtmenge der organischen Filtersubstanzen z.B. 1 bis 300 Gew.-%, bevorzugt 10 bis 250 Gew.-%, besonders bevorzugt 50 bis 200 Gew.-%, bezogen auf den Feststoffgehalt der Mischung.

Beispielsweise sind zu nennen:

**Tabelle 1:**

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfo)benzyliden-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 2,4,6-Trianilin-(o-carbo-21-ethylhexyl-1'-oxy)-1,3,5-triazin | 88122-99-0 |
| 18 | 3-Imidazol-4-yl-acrylsäure und ihr Ethylester | 104-98-3 |
| 19 | Menthyl-o-aminobenzoate oder: 5-Methyl-2-(1-methylethyl)-2-aminobenzoate | 134-98-3 |
| 20 | Glyceryl p-aminobenzoat oder: 4-Aminobenzoesäure-1-glyceryl-ester | 136-44-7 |
| 21 | 2,2'-Dihydroxy-4-methoxybenzophenon (Dioxy-benzone) | 131-53-3 |
| 22 | 2-Hydroxy-4-methoxy-4-methylbenzophenon (Mexenon) | 1641-17-4 |
| 23 | Triethanolamin Salicylat | 2174-16-5 |
| 24 | Dimethoxyphenylglyoxalsäure oder: 3,4-dimethoxy-phenyl-glyoxal-saures Natrium | 4732-70-1 |
| 25 | 3-(4'Sulfo)benzyliden-bornan-2-on und seine Salze | 56039-58-8 |
| 26 | 2,2',4,4'-Tetrahydroxybenzophenon | 131-55-5 |
| 27 | 2,2'-Methylen-bis-[6(2H-benzotriazol-2-yl)-4-(1,1,3,3,-tetramethylbutyl)phenol] | 103597-45-1 |
| 28 | 2,2'-(1,4-Phenylen)-bis-1H-benzimidazol-4,6-di-sulfonsäure, Na-Salz | 180898-37-7 |
| 29 | 2,4-bis-[4-(2-Ethylhexyloxy)-2-hydroxy]phenyl-6-(4-methoxyphenyl)-(1,3,5)-triazin | 187393-00-6 |
| 30 | 3-(4-Methylbenzyliden)-campher | 36861-47-9 |
| 31 | 4-Bis(polyethoxy)paraaminobenzoesäurepoly-ethoxyethylester | 113010-52-9 |
| 32 | 2,4-Dihydroxybenzophenon | 131-56-6 |
| 33 | 2,2'-Dihydroxy-4,4'-dimethoxybenzophenon-5,5'-dinatriumsulfonat | 3121-60-6 |

Weitere kombinierbare Lichtschutzmittel sind u.a. folgende, in WO 94/05645 und EP-A-0 444 323 beschriebene Verbindungen:

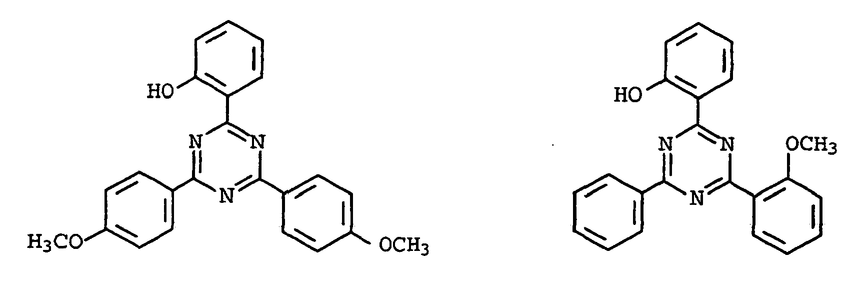

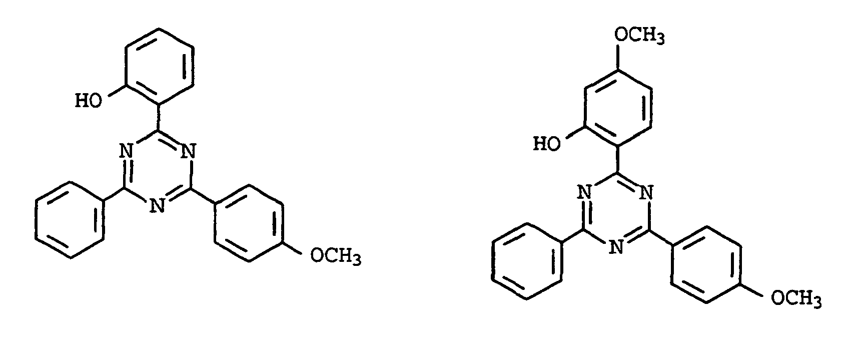

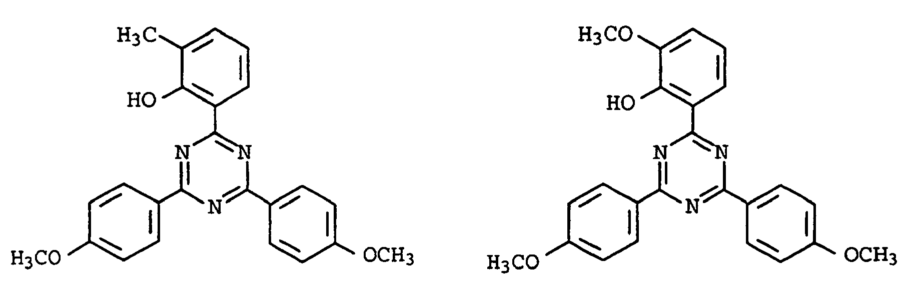

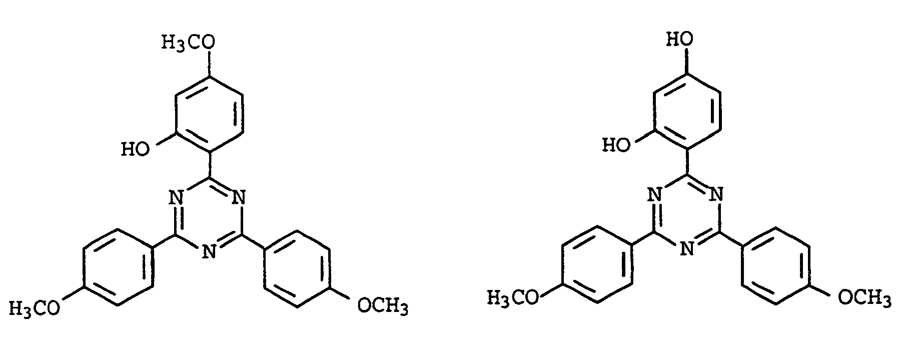

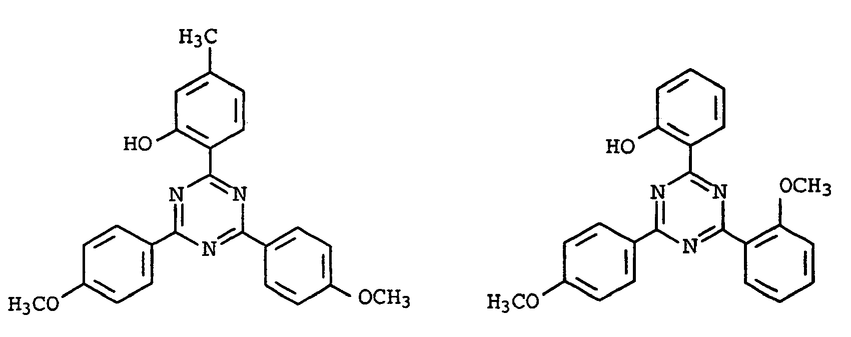

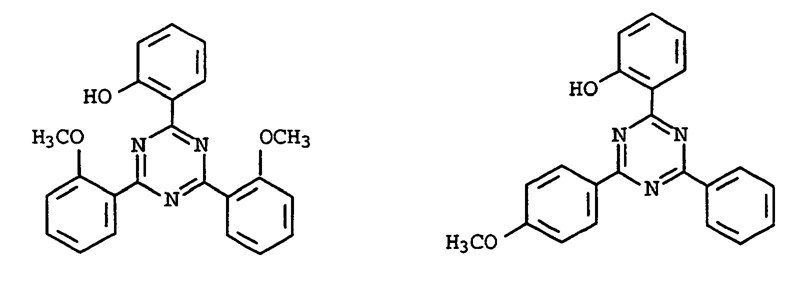

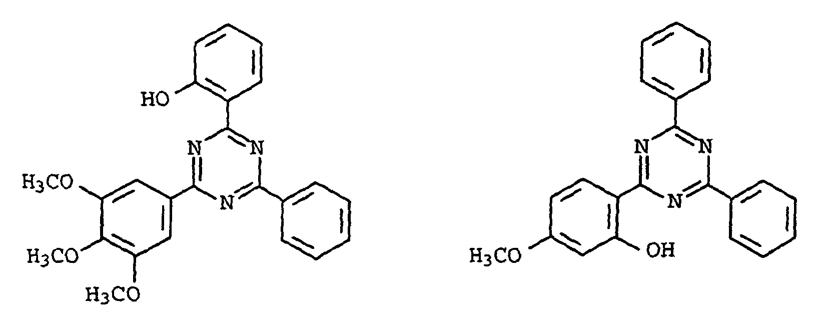

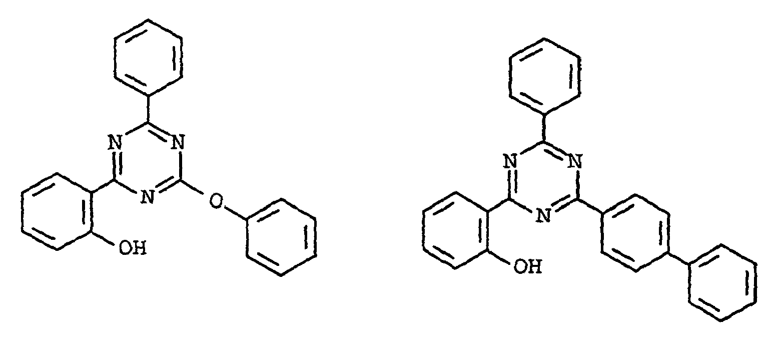

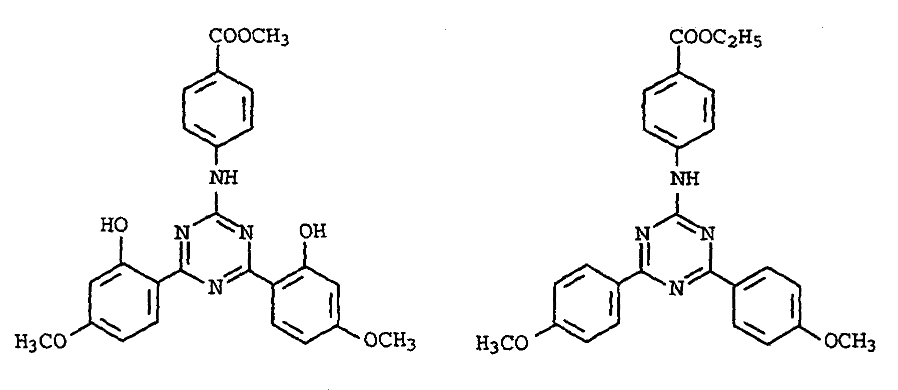

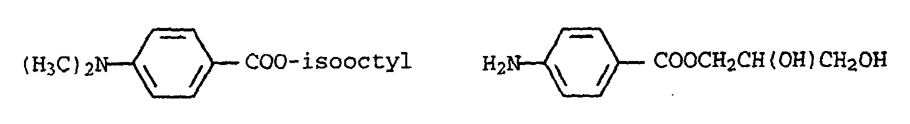

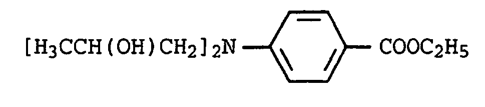

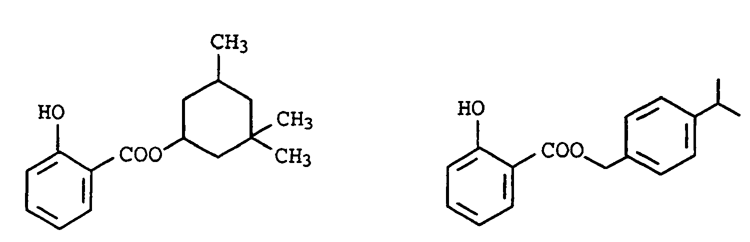

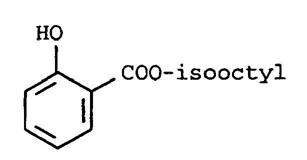

Die Liste der genannten UV-Filter, die in Kombination mit den erfindungsgemäßen Wirkstoffkombinationen verwendet werden können, soll selbstverständlich nicht limitierend sein.

Die erfindungsgemäßen Mischungen eignen sich als Ausgangsstoffe zur Herstellung kosmetischer und dermatologischer Zubereitungen.

Gegenstand der Erfindung ist daher auch insbesondere die Verwendung der eingangs genannten Mischungen als photostabile UV-Filter in kosmetischen und dermatologischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

Bevorzugt ist die Verwendung in Hautpflegemitteln, Lichtschutzmitteln, Antiaknemitteln, Make-up, Maskara, Lippenstifte, Lidschatten, Kajalstiften, Eyelinern, Rouges, Pudern und Augenbrauenstiften.

Gegenstand der Erfindung sind außerdem kosmetische und dermatologische Lichtschutzmittelzubereitungen zum Schutz der menschlichen Haut oder menschlichen Haare gegen Sonnenstrahlen, enthaltend die eingangs genannte Mischung.

Die Hautpflege- bzw. Lichtschutzmittelzubereitungen liegen insbesondere als W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen vor.

In den kosmetischen und dermatologischen Zubereitungen können die erfindungsgemäßen Mischungen besondere Wirkungen enfalten. Neben den UV-Strahlen-absorbierenden/-reflektierenden Eigenschaften der anorganischen UV-Filter (Komponente B) können die Polymere (Komponente A) unter anderem zur Feuchthaltung und Konditionierung der Haut und zur Verbesserung des Hautgefühls beitragen. Durch Zusatz der erfindungsgemäßen Polymere kann in bestimmten Formulierungen eine erhebliche Verbesserung der Hautverträglichkeit erreicht werden.

Die Polymere (A) bewirken ferner eine Stabilisierung kosmetischer und dermatologischer Zubereitungen, insbesondere von Emulsionen, die Pigmente anorganischer UV-Absorber enthalten.

Ein weiterer Vorteil der erfindungsgemäßen Mischungen ist das Erreichen höherer Sonnenschutzfaktoren in den kosmetischen und dermatologischen Formulierungen. Bei gleicher Menge an eingesetzen anorganischen UV-Filtern (B), führt der Zusatz der kationischen Polymere (A) zu einer Erhöhung des Sonnenschutzfaktors um mindestens den Faktor 1,1 bis 3,0, bevorzugt 1,1 bis 2,0, besonders bevorzugt 1,2 bis 1,5 im Vergleich zu Zubereitungen ohne (A).

Die erfindungsgemäßen Mischungen sind in den hautkosmetischen und dermatologischen Zubereitungen in einem Anteil von etwa 0,001 bis 30 Gew.-%, bevorzugt 0,01 bis 25 Gew.-%, besonders bevorzugt 0,1 bis 20 Gew.-%, ganz besonders bevorzugt 1 bis 15 Gew.-%, bezogen auf das Gesamtgewicht der Zubereitung, enthalten.

Die hautkosmetischen Zubereitungen können neben den erfindungsgemäßen Mischungen und geeigneten Lösungsmitteln noch in der Kosmetik übliche Zusätze, wie Emulgatoren, Konservierungsmittel, Parfümöle, kosmetische Wirkstoffe wie Phytantriol, Vitamin A, E und C, Retinol, Bisabolol, Panthenol, Bleichmittel, Färbemittel, Tönungsmittel, Bräunungsmittel (z.B. Dihydroxyaceton), Collagen, Eiweißhydrolysate, Stabilisatoren, pH-Wert-Regulatoren, Farbstoffe, Salze, Verdicker, Gelbildner, Konsistenzgeber, Silikone, Feuchthaltemittel, Rückfetter und weitere übliche Additive enthalten.

Als geeignete Lösungsmittel sind insbesondere zu nennen Wasser und niedrige Monoalkohole oder Polyole mit 1 bis 6 Kohlenstoffatomen oder Mischungen davon; bevorzugte Monoalkohole oder Polyole sind Ethanol, i-Propanol, Propylenglycol, Glycerin und Sorbit.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der oben genannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Übliche Verdickungsmittel in derartigen Formulierungen sind vernetzte Polyacrylsäuren und deren Derivate, Polysaccharide wie Xanthan-Gum, Agar-Agar, Alginate oder Tylosen, Carboxymethylcellulose oder Hydroxycarboxymethylcellulose, Fettalkohole, Monoglyceride und Fettsäuren, Polyvinylakolhol und Polyvinylpyrrolidon.

Man kann die erfindungsgemäßen Mischungen auch mit herkömmlichen Polymeren abmischen, falls spezielle Eigenschaften eingestellt werden sollen.

Als herkömmliche Polymere eignen sich beispielsweise anionische, kationische, amphotere und neutrale Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus t-Butylacrylat, Ethylacrylat, Methacrylsäure (z.B. Luvimer^{®} 100P), Copolymere aus Ethylacrylat und Methacrylsäure (z.B. Luvimer^{®} MAE), Copolymere aus N-tert.Butylacrylamid, Ethylacrylat, Acrylsäure (Ultrahold^{®} 8, strong), Copolymere aus Vinylacetat, Crotonsäure und gegebenenfalls weitere Vinylester (z.B. Luviset^{©} Marken), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol^{®} VBM), Copolymere von Acrylsäure und Methacrylsäure mit hydrophoben Monomeren, wie z.B. C₄-C₃₀-Alkylester der Meth(acrylsäure), C₄-C₃₀-Alkylvinylester, C₄-C₃₀-Alkylvinylether und Hyaluronsäure.

Als weitere Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane oder Silikonharze.

Die erfindungsgemäßen Mischungen werden in kosmetischen oder dermatologischen Zubereitungen eingesetzt, deren Herstellung nach den üblichen dem Fachmann geläufigen Regeln erfolgt.

Solche Formulierungen liegen vorteilhafterweise in Form von Emulsionen bevorzugt als Wasser-in-Öl-(W/O)- oder Öl-in-Wasser(O/W)-Emulsionen vor. Es ist aber auch erfindungsgemäß möglich und gegebenenfalls vorteilhaft andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen usw.

Die Herstellung erfindungsgemäß brauchbarer Emulsionen erfolgt nach bekannten Methoden.

Die Emulsionen enthalten neben dem erfindungsgemäßen Mischungen übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser.

Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, Dritter Teil, worauf hiermit ausdrücklich Bezug genommen wird.

So kann eine erfindungsgemäß brauchbare Hautcreme z.B. als W/O-Emulsion vorliegen. Eine derartige Emulsion enthält eine wäßrige Phase, die mittels eines geeigneten Emulgatorsystems in einer Öl- oder Fettphase emulgiert ist.

Die Konzentration des Emulgatorsystems beträgt in diesem Emulsions-Typ etwa 4 und 35 Gew.-%, bezogen auf das Gesamtgewicht der Emulsion; die Fettphase macht etwa 20 und 60 Gew.-% aus und die wäßrige Phasen etwa 20 und 70 Gew.-%, jeweils bezogen auf das Gesamtgewicht der Emulsion. Bei den Emulgatoren handelt es sich um diejenigen, welche in diesem Emulsionstyp üblicherweise verwendet werden. Sie werden z.B. ausgewählt unter: C₁₂-C₁₈-Sorbitan-Fettsäureestern; Estern von Hydroxystearinsäure und C₁₂-C₃₀-Fettalkoholen; Mono- und Diestern von C₁₂-C₁₈-Fettsäuren und Glyzerin oder Polyglyzerin; Kondensaten von Ethylenoxid und Propylenglycolen; oxypropylenierten/oxyethylenierten C₁₂-C₂₀-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink- oder Aluminiumlanolat und hydriertem Lanolin oder Lanolinalkohol.

Zu geeigneten Fettkomponenten, welche in der Fettphase der Emulsionen enthalten sein können, zählen Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karite-Öl, Hoplostethus-Öl; mineralische Öle, deren Destillationsbeginn unter Atmosphärendruck bei ca. 250°C und deren Destillationsendpunkt bei 410°C liegt, wie z.B. Vaselinöl; Ester gesättigter oder ungesättigter Fettsäuren, wie Alkylmyristate, z.B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Silikonöle, wie Dimethylpolysiloxan, Methylphenylpolysiloxan und das Silikonglycol-Copolymer, Fettsäuren und Fettalkohole enthalten.

Um die Retention von Ölen zu begünstigen, kann man auch Wachse verwenden, wie z.B. Carnauba-Wachs, Candellilawachs, Bienenwachs, mikrokristallines Wachs, Ozokeritwachs und Ca-, Mg- und Al-Oleate, -Myristate, -Linoleate und -Stearate.

Im allgemeinen werden diese Wasser-in-Öl-Emulsionen so hergestellt, daß die Fettphase und der Emulgator in den Ansatzbehälter gegeben werden. Man erwärmt diesen bei einer Temperatur von 70 bis 75°C, gibt dann die in Öl löslichen Ingredienzien zu und fügt unter Rühren Wasser hinzu, welches vorher auf die gleiche Temperatur erwärmt wurde und worin man die wasserlöslichen Ingredienzien vorher gelöst hat; man rührt, bis man eine Emulsion der gewünschten Feinheit hat, läßt sie dann auf Raumtemperatur abkühlen, wobei gegebenenfalls weniger gerührt wird.

Weiterhin kann eine erfindungsgemäße Pflegeemulsion als O/W-Emulsion vorliegen. Eine derartige Emulsion enthält üblicherweise eine Ölphase, Emulgatoren, die die Ölphase in der Wasserphase stabilisieren, und eine wäßrige Phase, die üblicherweise verdickt vorliegt.

Die wäßrige Phase der O/W-Emulsion der erfindungsgemäßen Zubereitungen enthält gegebenenfalls
- Alkohole, Diole oder Polyole sowie deren Ether, vorzugsweise Ethanol, Isopropanol, Propylenglycol, Glycerin, Ethylenglycolmonoethylether;
- übliche Verdickungsmittel bzw. Gelbildner, wie z.B. vernetzte Polyacrylsäuren und deren Derivate, Polysaccharide wie Xanthan Gum oder Alginate, Carboxymethylcellulose oder Hydroxycarboxymethylcellulose, Fettalkohole, Polyvinylalkohol und Polyvinylpyrrolidon.

Die Herstellung kann durch Aufschmelzen der Ölphase bei ca. 80°C erfolgen; die wasserlöslichen Bestandteile werden in heißem Wasser gelöst, langsam und unter Rühren zur Ölphase zugegeben; homogenisiert und kaltgerührt.

### A Herstellung der Polymere

### Herstellungsbeispiel 1

In einer Rührapparatur wurden 400 g Wasser und 46 g Dimethyldiallylammoniumchlorid-Lösung (65%ig) vorgelegt. Zu dieser Vorlage wurde 10% von Zulauf 1, bestehend aus 270 g N-Vinylpyrrolidon und 0,6 g N,N'-Divinylethylenharnstoff, gegeben. Unter Rühren im Stickstoffstrom wurde auf 60°C aufgeheizt und Zulauf 1 während 3 Stunden sowie Zulauf 2, bestehend aus 0,9 g 2,2'-Azo-bis(2-amidinopropan)dihydrochlorid in 100 g Wasser, während 4 Stunden zudosiert. Nach 3 Stunden verdünnte man mit 700 g Wasser und ließ eine weitere Stunde rühren. Danach gab man 1,5 g 2,2'-Azo-bis(2-amidinopropan)dihydrochlorid in 30 g Wasser zu und ließ weitere 2 Stunden bei 60°C rühren. Man erhielt eine farblose hochviskose Polymerlösung mit einem Feststoffgehalt von 20,9% und einem K-Wert von 80,3.

### Herstellungsbeispiel 2

In einer Rührapparatur wurden 300 g von Zulauf 1, bestehend aus 200 g N-Vinylpyrrolidon, 77 g Dimethyldiallylammoniumchlorid-Lösung (65%ig), 1,13 g N,N'-Divinylethylenharnstoff und 440 g Wasser, vorgelegt und unter Rühren im Stickstoffstrom auf 60°C aufgeheizt. Der Rest von Zulauf 1 wurde in 2 Stunden und Zulauf 2, bestehend aus 0,75 g 2,2'-Azo-bis(2-amidinopropan) dihydrochlorid in 100 g Wasser, in 4 Stunden zudosiert. Nach Ende von Zulauf 1 wurde das Reaktionsgemisch mit 1620 g Wasser verdünnt. Nach Ende von Zulauf 2 ließ man eine weitere Stunde bei 60°C rühren, gab danach 1,25 g 2,2'-Azo-bis(2-amidinopropan)dihydrochlorid in 65 g Wasser zu und rührte eine weitere Stunde. Man erhielt eine farblose hochviskose Polymerlösung mit einem Feststoffgehalt von 10,2%. und einem K-Wert von 80.

### Herstellungsbeispiel 3

In einer Rührapparatur wurden 130 g Wasser und 48 g 3-Methyl-1-vinylimidazoliumchlorid vorgelegt und unter Rühren im Stickstoffstrom auf 60°C aufgeheizt. Danach wurden Zulauf 1, bestehend aus 192 g N-Vinylpyrrolidon, 0,48 g N,N'-Divinylethylenharnstoff und 450 g Wasser, in 3 Stunden und Zulauf 2, bestehend aus 1,44 g 2,2'-Azo-bis(2-amidinopropan)dihydrochlorid in 80 g Wasser, während 4 Stunden zudosiert. Anschließend wurde noch eine Stunde bei 60°C gerührt. Um den Ansatz rührfähig zu halten, wurde nach Bedarf mit insgesamt 2100 g Wasser verdünnt. Man erhielt eine farblose hochviskose Polymerlösung mit einem Feststoffgehalt von 8,2% und einem K-Wert von 105.

### Herstellungsbeispiel 4

In einer Rührapparatur wurden 716 g Wasser vorgelegt und unter Rühren im Stickstoffstrom auf 60°C aufgeheizt. Danach wurde Zulauf 1, bestehend aus 180 g N-Vinylpyrrolidon, 20 g 3-Methyl-1-vinylimidazoliummethylsulfat, 0,32 g N,N'-Divinylethylenharnstoff und 25 g Wasser, in 2 Stunden und Zulauf 2, bestehend aus 0,6 g 2,2'-Azo-bis(2-amidinopropan)dihydrochlorid in 60 g Wasser, in 3 Stunden zudosiert. Nach Ende von Zulauf 1 wurde das Reaktionsgemisch mit 1000 g Wasser verdünnt. Im Anschluß an Zulauf 2 wurde noch 3 Stunden bei 70°C gerührt. Man erhielt eine farblose hochviskose Polymerlösung mit einem Feststoffgehalt von 11,0% und einem K-Wert von 86.

### Herstellungsbeispiel 5

In einer Rührapparatur wurden 440 g Wasser vorgelegt und unter Rühren im Stickstoffstrom auf 60°C aufgeheizt. Danach wurde Zulauf 1, bestehend aus 180 g N-Vinylpyrrolidon, 20 g 3-Methyl-1-vinylimidazoliummethylsulfat, 0,30 g N,N'-Divinylethylenharnstoff und 25 g Wasser, in 2 Stunden und Zulauf 2, bestehend aus 0,6 g 2,2'-Azo-bis(2-amidinopropan)dihydrochlorid in 60 g Wasser, in 3 Stunden zudosiert. Im Anschluß an Zulauf 2 wurde noch 3 Stunden bei 70°C gerührt. Um das Reaktionsgemisch rührfähig zu halten, wurde bei Bedarf insgesamt mit 1275 g Wasser verdünnt. Man erhielt eine farblose hochviskose Polymerlösung mit einem Feststoffgehalt von 11,3% und einem K-Wert von 105.

### Herstellungsbeispiel 6

In einer Rührapparatur wurden 650 g Wasser vorgelegt und unter Rühren im Stickstoffstrom auf 60°C aufgeheizt. Danach wurde Zulauf 1, bestehend aus 225 g N-Vinylpyrrolidon, 25 g 2,3-Dimethyl-1-vinylimidazoliummethylsulfat, 0,25 g N,N'-Divinylethylenharnstoff und 580 g Wasser, in 3 Stunden und Zulauf 2, bestehend aus 0,7 g 2,2'-Azo-bis(2-amidinopropan)dihydrochlorid in 100 g Wasser, in 4 Stunden zudosiert. Nach Ende von Zulauf 1 wurde das Reaktionsgemisch mit 835 g Wasser verdünnt. Im Anschluß an Zulauf 2 wurde noch eine Stunde gerührt und 1,25 g 2,2'-Azo-bis(2-amidinopropan)dihydrochlorid in 77 g Wasser nachdosiert. Danach rührte man noch 2 Stunden bei 70°C. Man erhielt eine farblose hochviskose Polymerlösung mit einem Feststoffgehalt von 10,4% und einem K-Wert von 106.

### Herstellungsbeispiel 7

In einer Rührapparatur wurden 650 g Wasser vorgelegt und unter Rühren im Stickstoffstrom auf 60°C aufgeheizt. Danach wurde Zulauf 1, bestehend aus 225 g N-Vinylpyrrolidon, 25 g 2,3-Dimethyl-1-vinylimidazoliummethylsulfat, 0,375 g N,N'-Divinylethylenharnstoff und 580 g Wasser, in 3 Stunden und Zulauf 2, bestehend aus 0,7 g 2,2'-Azo-bis(2-amidinopropan)dihydrochlorid in 100 g Wasser, während 4 Stunden zudosiert. Nach Ende von Zulauf 1 wurde das Reaktionsgemisch mit 1135 g Wasser verdünnt. Im Anschluß an Zulauf 2 wurde noch eine Stunde gerührt und 1,25 g 2,2'-Azo-bis(2-amidinopropan)dihydrochlorid in 77 g Wasser nachdosiert. Danach rührte man noch 2 Stunden bei 70°C. Man erhielt eine farblose hochviskose Polymerlösung mit einem Feststoffgehalt von 9,2% und einem K-Wert von 92.

### Herstellungsbeispiel 8

In einer Rührapparatur wurden 440 g Wasser vorgelegt und unter Rühren im Stickstoffstrom auf 60°C aufgeheizt. Danach wurde Zulauf 1, bestehend aus 144 g N-Vinylpyrrolidon, 16 g 3-Methyl-1-vinylimidazoliummethylsulfat, 1,4 g Tetraethylenglykoldiacrylat und 100 g Wasser, in 2 Stunden und Zulauf 2, bestehend aus 0,8 g 2,2'-Azo-bis(2-amidinopropan)dihydrochlorid in 50 g Wasser, in 3 Stunden zudosiert. Im Anschluß an Zulauf 2 wurde noch 3 Stunden bei 70°C gerührt. Um das Reaktionsgemisch rührfähig zu halten, wurde bei Bedarf insgesamt mit 1200 g Wasser verdünnt. Man erhielt eine farblose, hochviskose Polymerlösung mit einem Feststoffgehalt von 8,5% und einem K-Wert von 95.

### Herstellungsbeispiel 9

In einer Rührapparatur wurden 550 g Wasser vorgelegt und unter Rühren im Stickstoffstrom auf 60°C aufgeheizt. Danach wurde Zulauf 1, bestehend aus 102 g N-Vinylpyrrolidon, 26 g 3-Methyl-1-vinylimidazoliummethylsulfat, 0,8 g Triallylamin und 100 g Wasser in 2 Stunden zudosiert. Zulauf 2, bestehend aus 0,6 g 2,2'-Azo-bis(2-amidinopropan)dihydrochlorid in 50 g Wasser, wurde in 3 Stunden zur Reaktionsmischung zugegeben. Im Anschluß an Zulauf 2 wurde noch 3 Stunden bei 70°C gerührt. Um das Reaktionsgemisch rührfähig zu halten, wurde bei Bedarf insgesamt mit 1000 g Wasser verdünnt. Man erhielt eine schwach gelbliche, hochviskose Polymerlösung mit einem Feststoffgehalt von 7,0% und einem K-Wert von 102.

### B Anwendungsbeispiele

### Anwendungsbeispiel 1: Sonnenschutzcreme (A)

Gemäß folgender Rezeptur wurde zunächst eine erfindungsgemäße Wasser/Öl-Cremeemulsion (Sonnenschutzcreme A) hergestellt:

| Zusatz | Gew.-% |
|---|---|
| Ceteareth-6 und Stearylalkohol | 1,0 |
| Ceteareth-25 | 2,0 |
| Glyceryl Stearat | 3,0 |
| Cetearylalkohol | 2,0 |
| Cetearyloctanoat | 2,0 |
| Uninul T150 (Octyltriazon) | 1,0 |
| Uvinul MC 80 (Octyl Methoxycinnamat) | 5,0 |
| Uvinul MBC 95 (4-Methylbenzyliden Campher) | 3,0 |
| Z-Cote HP-1 (Zinkoxid) | 5,0 |
| Isopropyl Myristat | 7,0 |
| D-Panthenol | 0,5 |
| 1,2-Propylenglykol | 5,0 |
| Polymer (Herstellungsbeispiel 9) | 0,5 |
| Xanthan Gum (2% in Wasser) | 15,0 |
| Tocopherol Acetat | 1,0 |
| Parfümöl | q.s. |
| Konservierungsmittel | q.s. |
| Wasser | ad 100 |
| | |

Lichtschutzfaktor: 20 (Bestimmt nach der Colipa Methode, beschrieben in Parfuem. Kosmet. (1994), 75(12), 856)

### Vergleichsbeispiel 1: Sonnenschutzcreme (B) - ohne Polymerzusatz

| Zusatz | Gew.-% |
|---|---|
| Ceteareth-6 und Stearylalkohol | 1,0 |
| Ceteareth-25 | 2,0 |
| Glyceryl Stearat | 3,0 |
| Cetearylalkohol | 2,0 |
| Cetearyloctanoat | 2,0 |
| Uninul T150 (Octyltriazon) | 1,0 |
| Uvinul MC 80 (Octyl Methoxycinnamat) | 5,00 |
| Uvinul MBC 95 (4-Methylbenzyliden Campher) | 3,00 |
| Z-Cote HP-1 (Zinkoxid) | 5,00 |
| Isopropyl Myristat | 7,00 |
| D-Panthenol | 0,50 |
| 1,2-Propylenglykol | 5,0 |
| Polymer (Herstellungsbeispiel 9) | - |
| Xanthan Gum (2% in Wasser) | 15,00 |
| Tocopherol Acetat | 1,00 |
| Parfümöl | q.s. |
| Konservierungsmittel | q.s. |
| Wasser | ad 100 |
| | |

Lichtschutzfaktor: 15 (Bestimmt nach der Colipa Methode, beschrieben in Parfuem. Kosmet. (1994), 75(12), 856)

### Anwendungsbeispiel 2: Sonnenschutzcreme (C)

Gemäß folgender Rezeptur wurde zunächst eine erfindungsgemäße Wasser/Öl-Cremeemulsion (Sonnenschutzcreme C) hergestellt:

| Zusatz | Gew.-% |
|---|---|
| Ceteareth-6 und Stearylalkohol | 1,0 |
| Ceteareth-25 | 2,0 |
| Glyceryl Stearat | 4,0 |
| Cetearylalkohol | 2,0 |
| Cetearyloctanoat | 2,0 |
| Uninul T150 (Octyltriazon) | 1,0 |
| Uvinul MC 80 (Octyl Methoxycinnamat) | 5,0 |
| Uvinul MBC 95 (4-Methylbenzyliden Campher) | 3,0 |
| Z-Cote HP-1 (Zinkoxid) | 5,0 |
| Isopropyl Myristat | 7,0 |
| Dimethicon | 1,0 |
| D-Panthenol | 0,5 |
| 1,2-Propylenglykol | 5,0 |
| Polymer (Herstellungsbeispiel 9) | 0,5 |
| EDTA | 0,2 |
| Tocopherol Acetat | 1,0 |
| Phenoxyethanol | 0,5 |
| Methyldibromoglutaronitril | q.s. |
| Wasser | ad 100 |
| | |

Die Formulierung war kolloidal stabil

### Vergleichsbeispiel 2: Sonnenschutzcreme (D) - ohne Polymerzusatz

| Zusatz | Gew.-% |
|---|---|
| Ceteareth-6 und Stearylalkohol | 1,0 |
| Ceteareth-25 | 2,0 |
| Glyceryl Stearat | 4,0 |
| Cetearylalkohol | 2,0 |
| Cetearyloctanoat | 2,0 |
| Uninul T150 (Octyltriazon) | 1,0 |
| Uvinul MC 80 (Octyl Methoxycinnamat) | 5,0 |
| Uvinul MBC 95 (4-Methylbenzyliden Campher) | 3,0 |
| Z-Cote HP-1 (Zinkoxid) | 5,0 |
| Isopropyl Myristat | 7,0 |
| Dimethicon | 1,0 |
| D-Panthenol | 0,5 |
| 1,2-Propylenglykol | 5,0 |
| Polymer (Herstellungsbeispiel 9) | - |
| EDTA | 0,2 |
| Tocopherol Acetat | 1,0 |
| Phenoxyethanol | 0,5 |
| Methyldibromoglutaronitril | q.s. |
| Wasser | ad 100 |
| | |

Die Formulierung war kolloidal instabil

## Patentansprüche

1. Mischung, enthaltend
A) mindestens ein Copolymer, das erhältlich ist durch
(i) radikalisch initiierte Lösungspolymerisation eines Monomerengemisches aus
(a) 0,01 bis 99,99 Gew.-% mindestens eines Monomers, ausgewählt aus der Gruppe, bestehend aus N-Vinylimidazolen und Diallylaminen, gegebenenfalls in partiell oder vollständig quaternisierter Form;
(b) 0,01 bis 99,99 Gew.-% mindestens eines von (a) verschiedenen neutralen oder basischen wasserlöslichen Monomers;
(c) 0 bis 50 Gew.-% mindestens einer ungesättigten Säure oder eines ungesättigten Anhydrids;
(d) 0 bis 50 Gew.-% mindestens eines von (a), (b) oder (c) verschiedenen radikalisch copolymerisierbaren Monomers; und
(e) 0,01 bis 10 Gew.-% mindestens eines als Vernetzer wirkenden Monomers mit mindestens zwei ethylenisch ungesättigten, nichtkonjugierten Doppelbindungen und
(ii) anschließende teilweise oder vollständige Quaternisierung oder Protonierung des Polymeren für den Fall, daß das Monomer (a) nicht oder nur partiell quaternisiert ist
und
B) mindestens einen anorganischen UV-Filter.

2. Mischung nach Anspruch 1, **dadurch gekennzeichnet, daß** als Monomer (a) mindestens ein N-Vinylimidazol-Derivat der allgemeinen Formel (I), 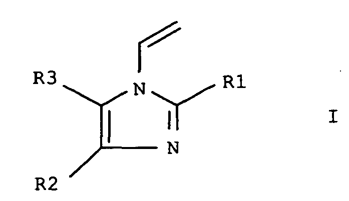 worin die Reste R¹ bis R³ unabhängig voneinander für Wasserstoff, C₁-C₄-Alkyl oder Phenyl stehen, verwendet wird.

3. Mischung nach Anspruch 1, **dadurch gekennzeichnet, daß** als Monomer (a) mindestens ein Diallylamin-Derivat der allgemeinen Formel (II), 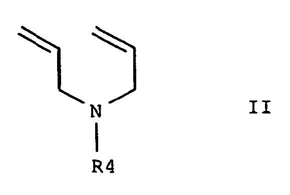 worin der Rest R⁴ für C₁-C₂₄-Alkyl steht, verwendet wird.

4. Mischung nach einem der Ansprüche 1 bis 3, **dadurch gekennzeichnet, daß** als Monomer (b) mindestens ein N-Vinyllactam verwendet wird.

5. Mischung nach einem der Ansprüche 1 bis 4, enthaltend als anorganischen UV-Filter B) mindestens ein mikronisiertes Metalloxid, ausgewählt aus der Gruppe, bestehend aus Titandioxid, Zinkoxid, Ceroxid, Aluminiumoxid, Siliciumdioxid, Zirkonoxid, Manganoxid, Aluminiumoxid und Eisenoxid.

6. Mischung nach Anspruch 5, enthaltend als anorganischen UV-Filter B) mindestens ein hydrophobiertes Metalloxid, ausgewählt aus der Gruppe, bestehend aus Titandioxid und Zinkoxid.

7. Mischung nach Anspruch 6, bei der das Metalloxid mit einem Silikon der allgemeinen Formel III, 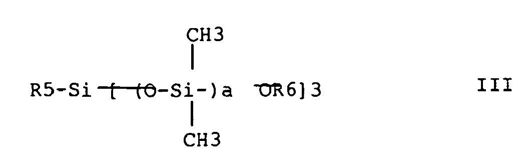 in der unabhängig voneinander R⁵ C₁-C₁₂-Alkyl und R⁶ Methyl oder Ethyl bedeuten und a für Werte von 4 bis 12 steht, gecoatet wurde.

8. Mischung nach einem der Ansprüche 1 bis 7, **dadurch gekennzeichnet, daß** der Anteil anorganischer UV-Filter 0,1 bis 99,9 Gew.-% beträgt.

9. Mischung nach einem der Ansprüche 1 bis 8, enthaltend mindestens einen weiteren organischen UVA- und/oder UVB-Filter.

10. Verwendung einer Mischung, definiert gemäß einem der Ansprüche 1 bis 9 zur Herstellung kosmetischer und dermatologischer Zubereitungen.

11. Verwendung nach Anspruch 10 als photostabile UV-Filter in kosmetischen und dermatologischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

12. Kosmetische und dermatologische Lichtschutzmittelzubereitungen zum Schutz der menschlichen Haut oder menschlichen Haare gegen Sonnenstrahlen, enthaltend eine Mischung, definiert gemäß einem der Ansprüche 1 bis 9.

## Claims

1. A mixture comprising
A) at least one copolymer obtainable by
(i) free-radically initiated solution polymerization of a monomer mixture of
(a) 0.01 to 99.99% by weight of at least one monomer chosen from the group consisting of N-vinylimidazoles and diallylamines, optionally in partially or completely quaternized form;
(b) 0.01 to 99.99% by weight of at least one neutral or basic water-soluble monomer which is different from (a);
(c) 0 to 50% by weight of at least one unsaturated acid or an unsaturated anhydride;
(d) 0 to 50% by weight of at least one free-radically copolymerizable monomer which is different from (a), (b) and (c); and
(e) 0.01 to 10% by weight of at least one monomer having at least two ethylenically unsaturated nonconjugated double bonds which acts as crosslinker, and
(ii) subsequent partial or complete quaternization or protonation of the polymer where the monomer (a) is not quaternized or only partially quaternized
and
B) at least one inorganic UV filter.

2. The mixture according to claim 1, wherein the monomer (a) used is at least one N-vinylimidazole derivative of the formula (I) 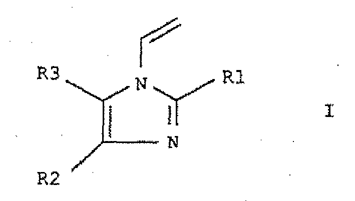 in which the radicals R¹ to R³, independently of one another, are hydrogen, C₁-C₄-alkyl or phenyl.

3. The mixture according to any of claims 1, wherein the monomer (a) used is at least one diallylamine derivative of the formula (II) 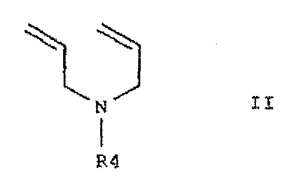 in which the radical R⁴ is C₁-C₂₄-alkyl.

4. The mixture according to any of claims 1 to 3, wherein the monomer (b) used is at least one N-vinyllactam.

5. The mixture according to any of claims 1 to 4, comprising, as inorganic UV filter B), at least one micronized mental oxide chosen from the group consisting of titanium dioxide, zinc oxide, cerium oxide, aluminum oxide, silicon oxide, zirconium oxide, manganese oxide, aluminum oxide and iron oxide.

6. The mixture according to claim 5, comprising, as inorganic UV filter B), at least one hydrophobicized metal oxide chosen from the group consisting of titanium dioxide and zinc oxide.

7. The mixture according to claim 6, in which the metal oxide has been coated with a silicone of the formula III 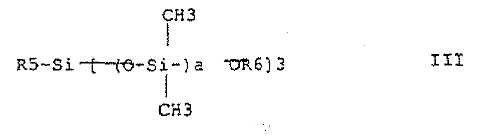 in which, independently of one another, R⁵ is C₁-C₁₂-alkyl and R⁶ is methyl or ethyl, and a is a value from 4 to 12.

8. The mixture according to any of claims 1 to 7, wherein the proportion of inorganic UV filters is 0.1 to 99.9% by weight.

9. The mixture according to any of claims 1 to 8, comprising at least one further organic UVA and/or UVB filter.

10. The use of a mixture defined as in any of claims 1 to 9 for the preparation of cosmetic and dermatological preparations.

11. The use according to claim 10 as photostable UV filter in cosmetic and dermatological preparations for protecting the human skin or human hair against solar rays, alone or together with compounds which absorb in the UV region and which are known per se for cosmetic and pharmaceutical preparations.

12. A cosmetic or dermatological sunscreen preparation for protecting the human skin or human hair against solar rays, comprising a mixture defined as in any of claims 1 to 9.

## Revendications

1. Mélange, contenant
(A) au moins un copolymère qui peut être obtenu par
(i) polymérisation en solution, amorcée par voie radicalaire, d'un mélange de monomères constitué de
(a) 0,01 à 99,99 % en poids d` au moins un monomère choisi dans l'ensemble constitué par les N-vinylimidazoles et les diallylamines, éventuellement sous forme partiellement ou totalement quaternaire ;
(b) 0,01 à 99,99 % en poids d'au moins un monomère hydrosoluble neutre ou basique, différent de (a) ;
(c) 0 à 50 % en poids d'au moins un acide insaturé ou d'un anhydride insaturé :
(d) 0 à 50 % en poids d'au moins un monomère copolymérisable par voie radicalaire, différent de (a), (b) ou (c) ; et
(e) 0,01 à 10 % en poids d'au moins un monomère agissant en tant qu'agent de réticulation, comportant au moins deux doubles liaisons à insaturation éthylénique non conjuguées et
(ii) quaternisation ou protonation partielle ou totale subséquente du polymère dans le cas où le monomère (a) n'est pas quaternaire ou ne l'est que partiellement,
et
(B) au moins un filtre UV inorganique.

2. Mélange selon la revendication 1, **caractérisé en ce qu'**on utilise comme monomère (a) au moins un dérivé de N-vinylimidazole de formule générale (I) 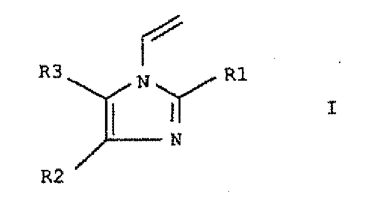 dans laquelle les radicaux R¹ à R³ représentent, indépendamment les uns des autres, un groupe alkyle en C₁-C₄ ou phényle.

3. Mélange selon la revendication 1, **caractérisé en ce qu'**on utilise comme monomère (a) au moins un dérivé de diallylamine de formule générale (II) 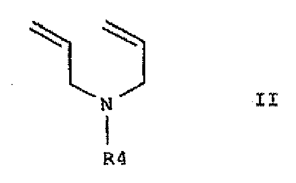 dans laquelle le radical R⁴ représente un groupe alkyle en C₁-C₂₄.

4. Mélange selon l'une quelconque des revendications 1 à 3, **caractérisé en ce qu'**on utilise comme monomère (b) au moins un N-vinyllactame.

5. Mélange selon l'une quelconque des revendications 1 à 4, contenant en tant que filtre UV inorganique (B) au moins un oxyde métallique micronisé, choisi dans l'ensemble constitué par le dioxyde de titane, l'oxyde de zinc, l'oxyde de cérium, l'oxyde d'aluminium, le dioxyde de silicium, l'oxyde de zirconium, l'oxyde de manganèse, l'oxyde d'aluminium et l'oxyde de fer.

6. Mélange selon la revendication 5, contenant en tant que filtre UV inorganique B) au moins un oxyde métallique hydrophobe, choisi dans l'ensemble constitué par le dioxyde de titane et l'oxyde de zinc.

7. Mélange selon la revendication 6, dans lequel l'oxyde métallique a été enrobé avec un silicone de formule générale III, 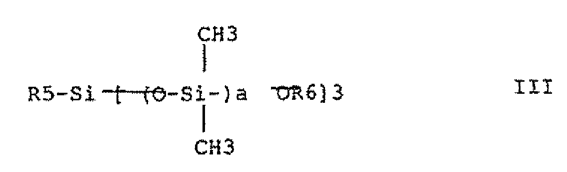 dans laquelle, indépendamment l'un de l'autre, R⁵ représente un groupe alkyle en C₁-C₁₂ et R⁶ représente un groupe méthyle ou éthyle, et a représente des valeurs allant de 4 à 12.

8. Mélange selon l'une quelconque des revendications 1 à 7, **caractérisé en ce que** la proportion des filtres UV inorganiques va de 0,1 à 99,9 % en poids.

9. Mélange selon l'une quelconque des revendications 1 à 8, contenant au moins un autre filtre UVA et/ou UVB organique.

10. Utilisation d'un mélange, défini selon l'une quelconque des revendications 1 à 9, pour la fabrication de préparations cosmétiques et dermatologiques.

11. Utilisation selon la revendication 10, en tant que filtre UV photostable dans des préparations cosmétiques et dermatologiques pour la protection de la peau humaine ou des cheveux humains contre les rayons du soleil, seul ou conjointement avec des composés absorbant dans la région UV, connus en soi pour des préparations cosmétiques et pharmaceutiques.

12. Préparations de photoprotecteurs cosmétiques et dermatologiques pour la protection de la peau humaine ou des cheveux humains contre les rayons du soleil, contenant un mélange selon l'une quelconque des revendications 1 à 9.
